# EUROPEAN PATENT SPECIFICATION

(11) **EP 3 856 170 B1**
(45) Date of publication and mention of the grant of the patent: **26.07.2023**
(21) Application number: 19791378.3
(22) Date of filing: 24.09.2019
(51) Int. Cl.: A61K 31/202, A61K 47/54, A61K 47/69, A61P 9/00

(54) **TREATMENT AND PROPHYLAXIS OF CARDIOVASCULAR DISORDERS WITH ERUCIC ACID**
BEHANDLUNG UND PROPHYLAXE VON KARDIOVASKULÄREN ERKRANKUNGEN MIT ERUCASÄURE
TRAITEMENT ET PROPHYLAXIE DE TROUBLES CARDIOVASCULAIRES AVEC DE L'ACIDE ÉRUCIQUE

(30) Priority: 26.09.2018 IT 201800008940
(43) Date of publication of application: 04.08.2021
(73) Proprietor: Università Degli Studi Di Verona, 37129 Verona (IT); Università Degli Studi Di Ferrara, 44121 Ferrara (IT)
(72) Inventor: MARTINELLI, Nicola, 37129 Verona, VR (IT); BERNARDI, Francesco, 44121 Ferrara (FE) (IT); BARONI, Marcello, 44121 Ferrara (FE) (IT); OLIVIERI, Oliviero, 37129 Verona (VR) (IT); GIRELLI, Domenico, 37129 Verona (VR) (IT); GUARINI, Patrizia, 37129 Verona (VR) (IT)
(74) Representative: Banchetti, Marina
(86) International application number: PCT/IT2019/050208
(87) International publication number: WO 2020/065690

(56) References cited:
- WO-A1-2011/060084
- AU-B2- 2010 363 574
- CHISA MATSUMOTO ET AL: "Red blood cell MUFAs and risk of coronary artery disease in the Physicians' Health Study", THE AMERICAN JOURNAL OF CLINICAL NUTRITION, vol. 98, no. 3, 3 July 2013 (2013-07-03), pages 749-754, XP055595429, US ISSN: 0002-9165, DOI: 10.3945/ajcn.113.059964
- G HORNSTRA ET AL: "Effect of the dietary fat type on arterial thrombosis tendency: systematic studies with a rat model", ATHEROSCLEROSIS, vol. 131, no. 1, 1 May 1997 (1997-05-01), pages 25-33, XP055594689, AMSTERDAM, NL ISSN: 0021-9150, DOI: 10.1016/S0021-9150(97)06094-2
- SAMIA HADJ AHMED ET AL: "Association of plasma fatty acid alteration with the severity of coronary artery disease lesions in Tunisian patients", LIPIDS IN HEALTH AND DISEASE, vol. 16, no. 1, 14 August 2017 (2017-08-14), XP055595476, DOI: 10.1186/s12944-017-0538-y

## Description

### Field of the invention

The present invention concerns erucic acid for use in the treatment and prevention of thrombotic disorders and cardiovascular diseases.

Herein also disclosed but not part of the invention is the determination of blood levels of erucic acid as a tool for diagnostic-prognostic classification of patients with thrombotic disorders and/or cardiovascular diseases. More particularly, the invention relates to the use of erucic acid (or c/s-13-docosenoic acid, 22:1 n-9) as a pharmaceutical or dietary supplement, to be administered, for example, carried in liposomes or other pharmaceutical formulations, for the prevention and treatment of coronary diseases, and for the prevention of the associated thrombotic disorders.

### Background of the invention

As is known, coronary heart disease and ischemic heart disease are pathologies responsible for an inadequate blood supply to the heart muscle; these conditions are generally caused by an accumulation of fat deposits within the arterial vascular walls. These deposits consist of cholesterol, calcium and other substances carried by the bloodstream, and the product of their accumulation is called atheroma or atherosclerotic plaque. The plaques can obstruct the coronary arteries and make them rigid and irregular, causing their hardening, known as atherosclerosis. Atherosclerosis is a complex pathophysiological process to which, first, chronic inflammatory mechanisms and alterations of lipid metabolism contribute.

These obstructions can present various levels of severity and appear at different sites while, as the disease progresses, the deposits restrict the lumen of the coronary arteries, thus reducing the supply of blood and oxygen to the cardiac muscle. This may cause chest pain (angina), breathing difficulties (dyspnea) and other symptoms. Complete obstruction may induce different types of heart attack (myocardial infarction, or ischemic myocardial necrosis) depending on the location and degree of obstruction, or an ischemic alteration with heart failure and arrhythmias.

Even if the degree of blood flow obstruction is not high, the atheroma may break abruptly, triggering the formation of blood clot (thrombus), which further narrows or totally obstructs the artery, giving rise to acute myocardial ischemia.

For the prophylaxis and therapy of coronary heart disease, a wide variety of drugs is generally used (in accordance with the multifactorial pathophysiology of atherothrombosis). These include platelet aggregation inhibitors and anti-coagulant drugs, such as acetylsalicylic acid, heparin, low molecular weight heparins, warfarin or direct-acting oral anticoagulants (DOAC), ACE inhibitors and beta-blockers, which are also indicated for the treatment of hypertension, nitroderivatives, such as nitroglycerin and isosorbide dinitrate or mononitrate, which are active in relieving arteries, fibrinolytic drugs such as streptokinase and rTPA, and cholesterol-lowering drugs, such as statins and fibrates.

In addition to pharmacological measures, of course, some lifestyle changes are considered important in the prevention of thrombotic and cardiovascular diseases, such as the elimination of smoking and the reduction of sedentary life, as well as changes in eating habits, with lower intake of saturated fats and greater intake of fruit, vegetables and fibers, moderate alcohol intake (if consumed), lower intake of simple carbohydrates (such as refined white flour, white rice, processed foods) and greater intake of foods based on whole grains.

In particular, to reduce the risk of coronary artery disease and thrombotic disorders, it is recommended to regularly take products with a high content of fish oils, such as salmon, high in omega-3 polyunsaturated fatty acids, and to strictly avoid the more harmful trans fatty acids. The latter have now been eliminated from the list of ingredients in many packaged food products and from foods found in fast-food menus and restaurants.

It is known that fatty acids are the main cellular lipids, which are much diversified based on the length of their chain and the number of double bonds present in them (i.e. the degree of desaturation). They not only represent the main constituents of the cell wall (phospholipids) and the energy fuel for cellular metabolism, but they can also modulate cellular functions through various pathways, acting as bioactive mediators, directly or through the synthesis of secondary messengers. Therefore, it does not surprise the fact that alterations in the profile of fatty acids in humans have been linked to the delicate balance between a healthy state and a pathological state.

In this context, so far most of the attention has been devoted, as already noted, to polyunsaturated fatty acids (PUFAs), and in particular to those of the omega-3 type, such as alpha-linolenic acid (ALA, 18:3 , n-3), eicosapentaenoic acid (EPA, 20:5, n-3) and docosahexaenoic acid (DHA, 22:6, n-3) and of the omega-6 type, such as linoleic acid (18:2, n-6) and gamma-linolenic acid (GLA, 18:3, n-6). The potentially favourable effects of the intake of these fatty acids have been studied extensively, and not only in the field of cardiovascular diseases: already in 2002 the American Heart Association recommended n-3 PUFAs for patients with ischemic heart disease and subjects with hypertriglyceridemia.

Further attention has been paid to the problem of trans fatty acids, such as elaidic or trans-9-octadecenoic acid (18:1 n-9). As already noted, trans fatty acids produced in the industrial processing of food are substances considered to be potentially harmful to health, and for several years now the US FDA has taken initiatives to reduce the intake of these substances in human food.

The available data on the effect on human health of other fatty acids than those of the categories mentioned above, on the other hand, are not as numerous, and, in addition, they are often contradictory to each other. In particular, the available data on erucic acid, a monounsaturated fatty acid with 22 carbon atoms (22:1, n-9, a superior homologue of oleic acid), and on its effect on human health are inconclusive in the current state and in some respects contradictory.

Studies conducted in the period between 1960 and 1980 on animal models showed that an excessive erucic acid intake can cause heart disease and cardiac lipidosis (Roine P et al. Z Ernahrungswiss 1960, 1:118-124; Schiefer B et al. Am J Pathol 1978, 90:551-564; Bremer J, et al. J Lipid Res 1982, 23:243-256; Van Vleet JF, et al. Am J Pathol 1986, 124:98-178). Further mechanistic studies have indicated that high levels of long-chain monoinsaturated fatty acids can damage the myocardium (Lopaschuk GD et al. Physiol Rev 2010, 90: 207-258; Goldberg Ira J et al. Cell Metabolism 2012; 15: 805-812).

As is known, heart preferably uses fatty acids as a fuel, but the mitochondria, which are the main energy-producing stations of cellular metabolism, lack transport enzymes through the membrane for long-chain fatty acids. Consequently, long chain fatty acids are predominantly oxidized in peroxisomes (Reddy JK et al., Ann Rev Nutr 2001, 21: 193-230). Unfortunately, peroxisomal oxidation produces reactive oxygen species (ROS) and several lipid metabolites, which inhibits mitochondrial oxidation and glycolysis, and promotes the synthesis of lipid droplets and the induction of apoptosis. This may eventually lead to significant cardiotoxicity, with induction of fibrosis and carboxy steatosis (Lopaschuk GD et al., Cell Metabolism 2012, 15: 805-812).

In accordance with the aforementioned concept of cardiotoxicity, a recent study has associated high levels of long-chain monounsaturated fatty acids, including erucic acid, with congestive heart failure incident in two independent US cohorts (Imamura F et al., Circulation 2013, 127: 1512-1521). However, an isolated study conducted within a cohort of US male physicians - the Physicians' Health Study - suggested an inverse association between erucid acid levels in red blood cells and ischemic cardiopathy, albeit without providing any further correlation with intermediate biological and/or cardiovascular phenotypes (Matsumoto C. et al., Am. J. Clin. Nutr. 2013; 98:749-54).

Based on these observations, erucic acid is generally considered to be a potentially toxic fatty acid, and for this reason several governments limit its content in edible oils. For this reason the food sources of erucic acids, such as rapeseed oil, have been critically considered, limited and modified (as it was the case, for example, for canola oil - low-erucic acid Canadian rapeseed oil, which contains less than 2% erucic acid) (Daun J. J Am Oil Chem Soc 1986, 63: 321-324; Food and Drug Administration USA, Fed Regist 1985, 50: 3746). Despite this, food composition data indicate that erucic acid remains regularly present in the western diet, being a component of many foods, such as mustard oil, some fish species as well as meat and poultry products (US Department of Agriculture, Agricultural Research Service, 2009, USDA National Nutrient Database for Standard Reference, Release 22, Nutrient Data Laboratory Home Page, http://www.ars.usda.gov/ba/bhnrc/ndl, Access March 20, 2015).

Despite this reputation as a harmful fatty acid, erucic acid was proposed in 1989 as a potential therapeutic tool in patients with X-linked adrenoleukodystrophy (X-ALD) (Rizzo WB et al., Neurology 1989, 39: 1415-1422). X-ALD is a peroxisomal disease due to the mutation of the ABCD1 gene that codes for a peroxisomal protein that carries very long chain fatty acids (VLCFA) in peroxisomes (Mosser J et al. Nature 1993; 361: 726-730). X-ALD is biochemically characterized by an accumulation of very long chain fatty acids (in particular 24:0, lignoceric acid and 26:0, cerotic acid) in plasma and tissues, and clinically by progressive demyelination and adrenal insufficiency (Berger J et al. Brain Pathol 2010; 20: 845-856). The so-called Lorenzo's oil, which is a mixture of 4 parts of glyceryl-trioleate and 1 part of glyceryltrierucate, can reduce plasma levels of saturated VLCFA in patients with adrenoleucodystrophy, probably by inhibiting the activity of the enzyme ELOVL1 (Sassa T et al. J Lipid Res 2014; 55: 524-530), but the clinical effects remained controversial.

Lorenzo's oil may slightly delay the onset of symptoms in asymptomatic patients, but it is ineffective when the symptoms have already deteriorated (Moser HW et al. J MolNeurosci 2007; 33: 105-113).

It should be noted that other potential effects of erucic acid at physiological levels have never been studied in humans.

Regarding the effect of fatty acids (other than erucic acid) on hemostasis, some of them have been studied in general due to their ability to modulate platelet activation and aggregation. In particular, arachidonic acid, one of the main n-6 PUFAs which is the precursor of a wide range of important eicosanoids, including thromboxanes (potent pro-aggregants), induces platelet aggregation in vitro, and is often used in laboratory tests on platelet function (Zhou L et al. Am J Clin Pathol 2005; 123: 172-183). On the other hand, food arachidonic acid does not produce significant changes in platelet function, as it does not produce significant changes in whole blood coagulation (Nelson GJ et al. Lipids 1997; 32: 421-425).

In contrast, PUFAs n-3 were considered able to attenuate platelet activation (Cohen MG et al. Thromb Res. 2011; 128: 335-40), while supplementation with PUFA n-6 was associated with a slight inhibition of platelet aggregation (Lev EI et al. J Am Coll Cardiol 2010; 55: 114-121) and a moderate reduction in the overall coagulation potential (McEwen BJ et al. Semin Thromb Haemost 2015; 41: 315-22). Overall, therefore, these results remain controversial (Gao LG et al. Atherosclerosis, 2013; 226: 328-34; Reiner MF et al. Thromb Haemost 2015; 113: 177-184) and in any case the overall effects on hemostasis seem to be modest (De Caterina R. N Engl J Med 2011; 364: 2439-2450).

However, the physiological effects of fatty acids in the haemostatic pathways may not be limited to platelet aggregation, and not be limited to the effects of PUFAs n-3. For example, phospholipids, which are the main components of cell membranes, have been recognized since long time as fundamental cofactors and as necessary for the initiation and propagation of the coagulation cascade (Rouser G. Am J Clin Nutr 1958; 6: 681-687). Furthermore, the tissue factor (TF), which plays a key role in the physiological activation of coagulation, requires the association with phospholipids to perform its biological activity. G. Hornstra et al, (Atherosclerosis, vol.113, no 1, 1997, pages 25-33) has reported that various fatty acids have either a prothrombotic effect or an anti-thrombotic effect in a rat model.

Returning specifically to erucic acid, a previous isolated *in vitro* study, which evaluated a wide range of natural compounds belonging to the classes of phenols and vegetable fatty acids, suggested that oleic and erucic unsaturated fatty acids may inhibit the enzymatic activity of thrombin, with potential positive effects in use as anti-inflammatory adjuvants in the inhibition of neutrophil elastase (Melzig MF et al. Planta Med 2005; 71: 787-789). Nevertheless, so far the relationship between fatty acids and coagulation has not been systematically investigated.

From the foregoing it is clear the interest in having a substance of natural origin, obtainable in a simple and economic way from plant sources, that lends itself to therapeutic and/or prophylactic use as a dietary supplement or as a drug or food supplement for the prevention and treatment of cardiovascular diseases such as coronary heart disease, and for the prophylaxis of thrombotic disorders. Such a product could be used in replacement of the currently used anticoagulant and antithrombotic agents or in addition to them, to reduce the incidence and consequences of these pathologies.

### Summary of the invention

In the frame of the studies connected with the present invention it has been found, contrary to what has been suggested by most of the literature mentioned above, that high blood levels of erucic acid (within physiological values) in the blood are associated with a reduced risk of mortality from cardiovascular diseases.

It is known that coagulation plays a crucial role in the development and complications of coronary artery diseases, as reported in the literature (Borissoff JI et al. N Engl J Med. 2011; 364: 1746-60), and that the hypercoagulability of blood may predispose to cardiovascular events, and also that increased generation of thrombin has been associated with coronary artery disease (Smid M et al. J Thromb Haemost. 2011; 9 (3): 450-6; Ten Cate H Thromb Res. 2012; 129 (3): 367-70; Smid M et al. PLoS One. 2013; 8 (6): e66977; Kalz J et al. J Thromb Thrombolysis. 2014; 37 (1): 45- 55). On the basis of these biological and epidemiological reasons the authors of the present invention have hypothesized that high levels of erucic acid in the blood, which they found to be correlated with a lower endogenous thrombin potential, could be associated with a reduced risk of coronary heart disease.

In confirmation of the hypothesis formulated, it has been found, according to the present invention, that the lack of erucic acid in blood plasma and red blood cells is associated with an increased risk of thrombotic disorders in coronary artery diseases, so that the levels of erucic acid were found to be an independent predictor of the endogenous thrombin potential (the latter is a global parameter of coagulation, recalling *inter alia* that thrombin is the final and principal effector of the coagulation cascade). It has also been found that erucic acid deficiency is associated with an increased mortality from cardiovascular events in patients known for ischemic heart disease, as shown by the experimental study presented below.

According to the present invention, the maintenance of physiologically elevated blood levels of erucic acid has favourable cardiovascular effects thanks to the anticoagulant properties of this acid, which is able to reduce the thrombin generation. For these reasons, the supplementation of erucic acid in the diet, or as a food supplement or nutraceutical, is proposed for the prevention and treatment of cardiovascular pathologies, and for prevention of thrombotic disorders, in addition to the normal lifestyle measures and possibly drugs indicated for coronary heart disease.

Since the studies conducted by the authors of the present invention have revealed that the subjects with the lowest levels of erucic acid in the blood are those with the highest risk of complications in coronary artery disease, this disclosure also suggests but does not claim, that diagnostic methods based on determining the concentration of erucic acid in plasma and/or in the erythrocytes may be used as a useful biomarker in coronary heart disease and in cardiovascular diseases in general. In such methods, the determination of a blood concentration of erucic acid that is too far, by default, from the concentration limits considered as physiologically protective in healthy subjects, may be taken as a risk index for thrombotic events for the patient.

### Brief description of the figures

The specific characteristics of the invention, as well as the advantages thereof, will be more evident with reference to the detailed description, to the experimental work reported below and to the relative figures, in which:
**Figures 1** **(A, B, C, D, E)** are respectively a histogram (A) showing the distribution in quartiles of an experimental population of subjects analysed for the plasma concentration (%) of erucic acid, divided between healthy subjects (CAD-free) and subjects with atherosclerotic coronary artery disease (CAD), and a diagram of the relative risk of CAD, unadjusted (B) and then adjusted (C, D, E) for potential confounders, for the different quartiles of Figure 1 ;
**Figures 2** **(A, B, C, D, E)** are respectively a histogram (A) showing the distribution in quartiles in the same population of Figure 1, with respect to the erythrocyte concentration (%) of erucic acid, of the same subjects, and a diagram of the relative risk of CAD, unadjusted (B) and then adjusted (C, D, E) for potential confounders, for the different quartiles of Figure 2;
**Figures 3A** and **3B** show the Kaplan-Meier survival curves respectively for total mortality and cardiovascular mortality in each of the four quartiles of plasma concentration of erucic acid in which the CAD population was divided in the study;
**Figures 4A** and **4B** show the Kaplan-Meier survival curves respectively for total mortality and cardiovascular mortality for the same subjects of Figures 3A and 3B divided into two groups: with plasma erucic acid higher than the median value (≥0.15%) or lower; and
**Figures 5** **(A, B, C, D, E, F)** show the diagrams of the thrombin generation test in a 1:4 mixture of normal commercial plasma and factor V depleted plasma, supplemented with liposomes containing erucic acid at two different concentrations, or not containing erucic acid.

### Detailed description of the invention

The present invention, therefore, specifically concerns a preparation for systemic administration based on erucic acid (cis-13-docosenoic acid, 22:1 n-9) for use as an anticoagulant and antithrombotic agent.

Preferably, according to an embodiment of the invention, said preparation is proposed for use in the prevention and treatment of cardiovascular diseases and thrombotic disorders, and in particular is proposed in the case in which said cardiovascular diseases are coronary artery diseases.

According to preferred embodiments of the invention, the preparation for use according to the invention is formulated in a vehicle also suitable for oral administration.

Lipophilic substances such as erucic acid often present administration difficulties due to their intrinsic characteristics, which limit their solubility in water and consequently limit their relative absorbability and bioavailability. These lipophilic substances are good candidates for the use of advanced solubilization technologies, such as solubilization by surfactants, the use of amorphous drugs, the use of encapsulation and/or complexation techniques (e.g.: cyclodextrins) and administration by liposomes.

In particular, a potential vehicle for ensuring an effective release of erucic acid in the bloodstream may be in the form of lipid micelles or liposomes containing erucic acid in their composition. More precisely, liposomes are vesicular lipid structures, with a diameter varying between 2.5 micrometres and 20 nanometres, consisting of one or more double layers, mainly phospholipids, which delimit one or more hydrophilic cores. In the external lipid layer the hydrophilic polar heads of the phospholipids are exposed, in the inner one the hydrophobic tails, are exposed in contact with the tails of the inner layer, which has a specular organization, with the polar heads turned towards the aqueous cavity of the liposome. This lipid envelope not only isolates and protects the hydrophilic content of the liposomes from the surrounding environment, but also allows it to be conveyed inside cells, in turn protected by a chemically quite similar - and therefore analogous - membrane. Also the lipid bilayer of the liposomes can be used to transport molecules, in this case hydrophobic molecules. Due to these characteristics, since the 1970s liposomes have been widely used in the medical, pharmaceutical and cosmetic fields, to convey molecules of various chemical nature for various purposes.

Liposomes very frequently consist of natural lipids (as erucic acid itself is), which considerably limits their ts toxic effects and the possible immune responses of the treated organism, but can also be created from synthetic lipids, in order to vary, even considerably, some basic characteristics of the micelles, from charge to stability. Furthermore, by incorporating specific molecules into the external liposomal membrane, said molecules being not only of a lipid nature, but also glucides or proteins (such as antibodies), it is possible to confer a high tissue-specificity to the liposome, making it a sophisticated, albeit economic in its large-scale synthesis, drug delivery system. A further advantage is the fact that it is possible to administer liposomes and molecules contained therein both orally and parenterally, and finally also topically, which makes them a particularly versatile molecular instrument. The main limitation of liposomes lies in their poor stability and tendency to degrade by oxidation. It is however possible, in order to also enormously increase their stability, to coat the liposomes with polyethylene glycol molecules, to hydrogenate, even partially, the unsaturated fatty acids present therein, to preserve the liposomes in the presence of antioxidants, and finally to freeze the liposomes.

According to a specific solution illustrated in the experimental part which follows, said preparation is formulated in a vehicle consisting of liposomes, for example liposomes made of phosphatidylcholine diesterified with stearic acid, and phosphatidylserine diesterified with stearic acid (preferably in a proportion of about 90(distearoyl-phosphatidylcholine):10(distearoyl-phosphatidylserine)) containing from 0.05% to 1.00% by weight of phosphatidylcholine diesterified with erucic acid. Preferably the dierucoyl-phosphatidylcholine content is between 0.1% and 0.5% by weight, and even more optimally this content in the liposomes is 0.5% by weight. As customary, after the production of liposomes, whatever the technique used, these are further treated (for example, by extrusion) to homogenize their diameter. The preferred diameter for liposomes for this application is approximately 200 nm.

Still according to the present invention, the erucic acid proposed for use as an antithrombotic and anticoagulant in patients with cardiopathies, and in particular in patients with coronary artery disease and subject to thrombotic disorders, should be administered at a daily dosage such as to restore the medium-high physiological blood concentrations of this acid. That is, concentrations higher than 0.30% with regard to the plasma or erythrocyte level (and in any case less than 1.00%). Therefore, according to this invention, erucic acid should be administered at a dosage of between 50 mg/day and 500 mg/day, preferably between 200 mg/day and 400 mg/day, until the above blood levels are restored.

The disclosure also suggests, as already noted, the adoption of a diagnostic-prognostic evaluation procedure in subjects with cardiovascular diseases, ischemic heart disease and thrombotic disorders, which includes the following operations:
- taking a blood sample from a subject, and obtaining the measure of blood levels of erucic acid from the blood sample, typically using a gas chromatographic method, and
- classifying said subject as one having an increased thrombophilic diathesis and an increased cardiovascular risk, and therefore likely to benefit from selective supplementation with erucic acid, if the blood levels of erucic acid are lower than 0.30% by weight.

The dosage of blood levels of erucic acid preferably includes both the plasma and erythrocyte levels.

### CLINICAL POPULATION STUDY IN SUBJECTS WITH CORONARY DISORDERS

### Study population

The analyses were performed in the Verona Heart Study (VHS), a regional survey aimed at detecting new risk factors for coronary artery disease in subjects with angiographic documentation on the status of their coronary arteries (Girelli D et al. N Engl J Med. 2000; 343: 774-780; Martinelli N, et al. Blood. 2010; 116: 5688-5697).

The subjects included in the study did not have to present stories of acute illness in the month preceding enrolment and, according to this criterion, patients with acute coronary syndrome were excluded from the study. Furthermore, subjects with severe renal insufficiency (estimated glomerular filtration rate (eGFR) <30 ml/min) and those with severe hepatic impairment (clinically defined diagnosis of liver cirrhosis) were also excluded from the study.

Coronary artery disease (CAD) patients presented at least one of the major epicardial coronary arteries (left anterior descending, circumflex, and right) affected with ≥1 significant stenosis (>50% lumen reduction). All the CAD patients were newly diagnosed at the time of enrolment, i.e. at the time of coronary angiography. The angiograms were assessed blind by two cardiologists who were unaware that the patients were to be included in this study.

Moreover, subjects with completely normal coronary arteries undergoing coronary angiography for reasons other than CAD (mainly valvular heart disease), were used as controls (CAD-free group). These subjects had no history, or clinical or instrumental evidence of atherosclerosis outside the coronary bed and none were taking any anti-platelet drugs.

All participants came from the same geographical area of northern Italy. At the time of blood sampling, a complete clinical history was collected, as well as data about drug therapies. The study complies with the Declaration of Helsinki and was approved by the Ethic Committee of our Institution (Azienda Ospedaliera Universitaria Integrata, Verona). A written informed consent was obtained from all the participants.

### Measurement of fatty acid concentrations in plasma and in red blood cell (RBC) plasmatic membrane

Blood samples were transported to the laboratory within 1 hour of collection and processed immediately. The analysis of fatty acids in the plasma (100 µl) and in the erythrocyte membrane (250 µl of concentrated hemolyzed erythrocytes an in equal volume of bidistilled water) was performed on the total lipids extracted using 4.5 mL of isopropanol in chloroform in ratio 11:7 by volume containing 0.45 mmoles 2,6-di-ter-p-cresol/L as antioxidant.

A gas chromatographic method (Hewlett-Packard 5980 chromatograph, equipped with a 25 m HP-FFAP column, 0.2 mm internal diameter, 0.3 µm phase; Hewlett-Packard, Palo Alto, CA) based on a direct FA transesterification technique has been used as described above (Martinelli N et al. Am J Clin Nutr 2008; 88: 941-949). The analyses were performed in duplicate on each sample.

Peak identification and quantification were performed with commercially available reference fatty acids (Sigma, St. Louis, MO). Margaric acid (heptadecanoic, 17:0) was used as an internal standard. Peak areas were measured and quantification was performed with the help of a Vectra QS/16S PC equipped with the HP-3365 CHEM STATION software (version 3.0, Hewlett-Packard).

The fatty acids were expressed as g/100 g of fatty acid methyl ester (% by weight). Fatty acids containing from 12 carbon atoms to 26 carbon atoms were measured, namely: saturated fatty acids: 12:0, 14:0, 16:0, 18:0, 20:0, 22:0, 24:0 and 26:0; monounsaturated fatty acids: 16:1, 18:1 n-9, 20:1 and 22:1; and polyunsaturated fatty acids (PUFA): 18:2 n-6, 18:3 n-3, 20:2 n-6, 20:4 n-6, 20:5 n-3, and 22:6 n-3. Unidentified peaks accounted for less than 0.5% of the total. All intra-assay and inter-assay variation coefficients were <5%.

### Thrombin generation assay

Thrombin generation was measured on frozen citrate plasma samples, never thawed before the study. Thrombin generation assay was determined using the Endogenous Thrombin Potential Test (ETP) (*for research use only,* Siemens Healthcare Diagnostics Products GmbH, Marburg, Germany). The conversion kinetics of a synthetic thrombin substrate was measured by the release of a chromophore in a platelet-poor plasma sample, at a wavelength of 405 nm in a chromogenic assay.

Thrombin formation began with the addition of recombinant tissue factor (Dade^{®} Innovin^{®} Reagent, Siemens Healthcare Diagnostics Products GmbH, Marburg, Germany) and calcium chloride. Four thrombin generation parameters were derived from the thrombin generation curve: i) lag time, ii) peak time, iii) peak height and iv) endogenous thrombin potential (ETP), whose value was derived from the area under the thrombin generation curve.

Finally, for the calibration of the ETP assay, an ETP standard was used, that is to say a normal plasma pool from selected healthy donors, which had been delipidated and subsequently calibrated for the normal percentage and normal U/mL of Factor II (these refer to fresh human citrated plasma pool exhibiting 100% of the norm or 1 U/ml for all factors by definition). All tests were performed in duplicate. The intra-assay and inter-assay coefficients were <5%.

### Results from bedside: the clinical significance of erucic acid plasma and red blood cell concentration

**Erucic acid is inversely correlated with thrombin generation and is an independent predictor of endogenous thrombin potential.**

Thrombin generation and plasma fatty acid profile test data were available for a total of 293 subjects (mean age 59.6 ± 11.4 years, 23.9% females), while fatty acid data in red blood cells were available for 292 subjects. All these subjects were not taking anticoagulant drugs at the time of blood collection for enrolment and did not have any acute illness in the previous three months. Correlations were assessed using Pearson's test. Logarithmic transformation for data analysis was performed on all the variables having a skewed distribution.

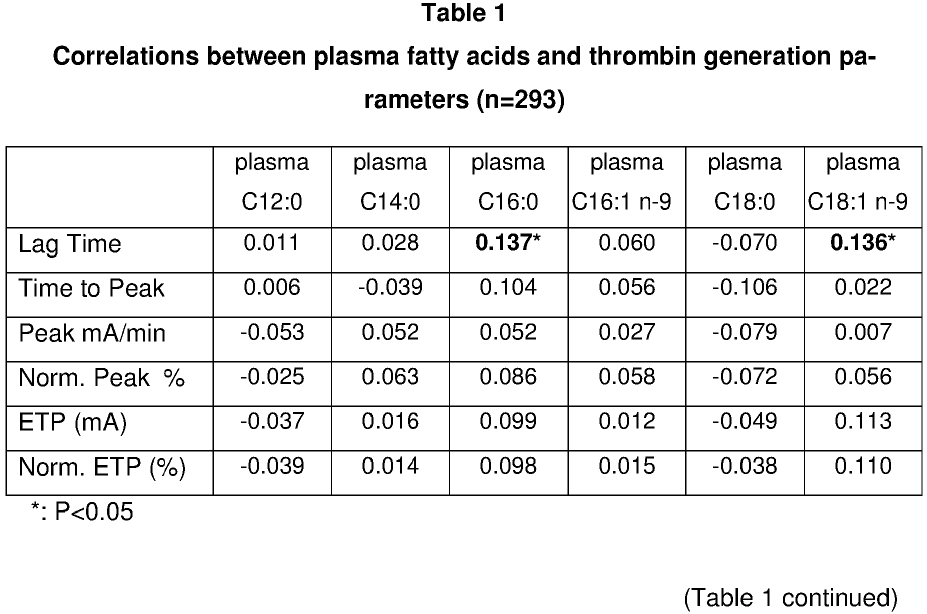

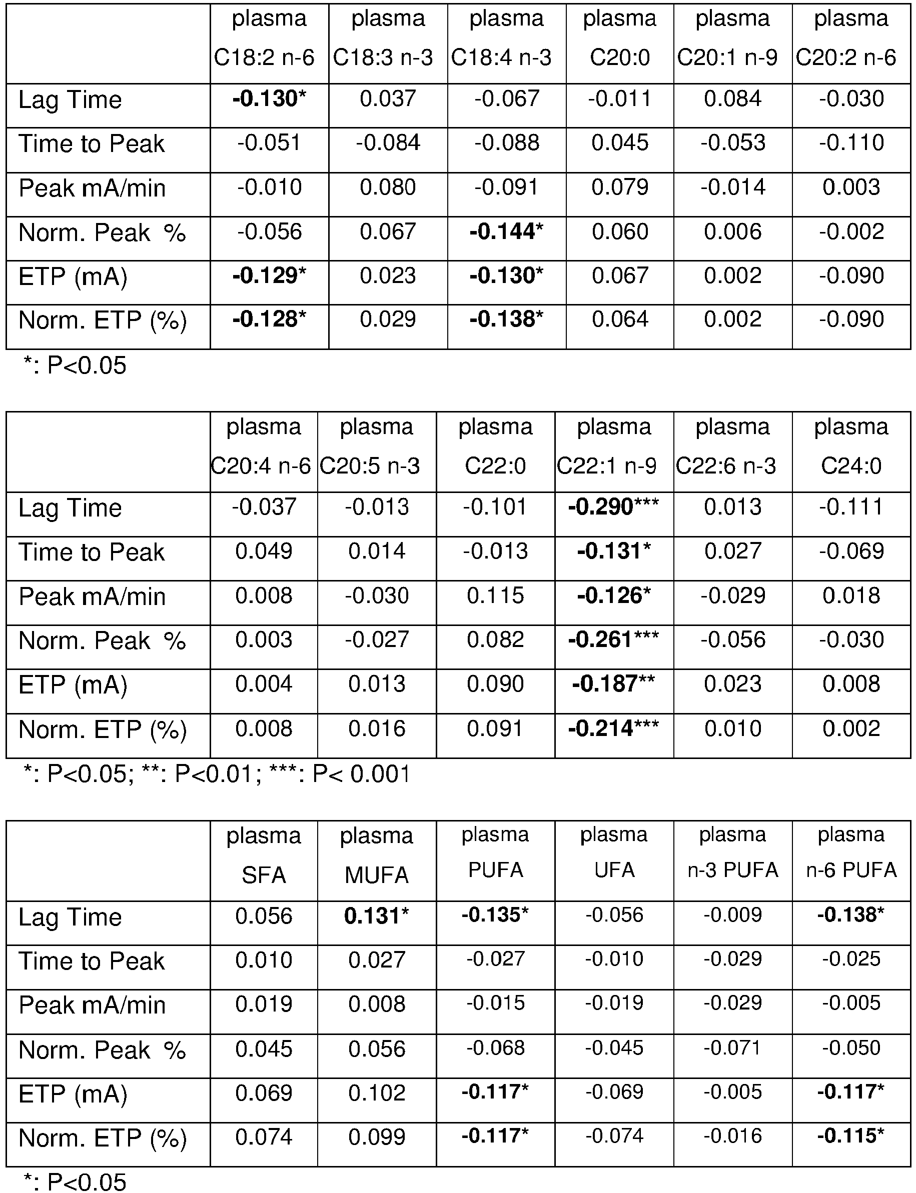

As can be seen from the table above, with regard to the analysis of plasma fatty acids, erucic acid had significant inverse correlations with all the thrombin generation parameters, often with high statistical significance (P <0.001), while the other fatty acids showed only a few mild correlations (P <0.05).

The significant inverse correlations between erucic acid and thrombin generation have been substantially confirmed by the analysis of fatty acids in red blood cells. However, as can be seen from the data reported in the following Table 2, in the latter analysis also other fatty acids showed significant correlations, more precisely stearidonic acid (18: 4 n-3 - inverse correlation), acid myristic (14:0 - inverse correlation), arachidic acid (20:0 - direct correlation), behenic acid (22:0 - direct correlation) and lignoceric acid (24:0 - direct correlation).

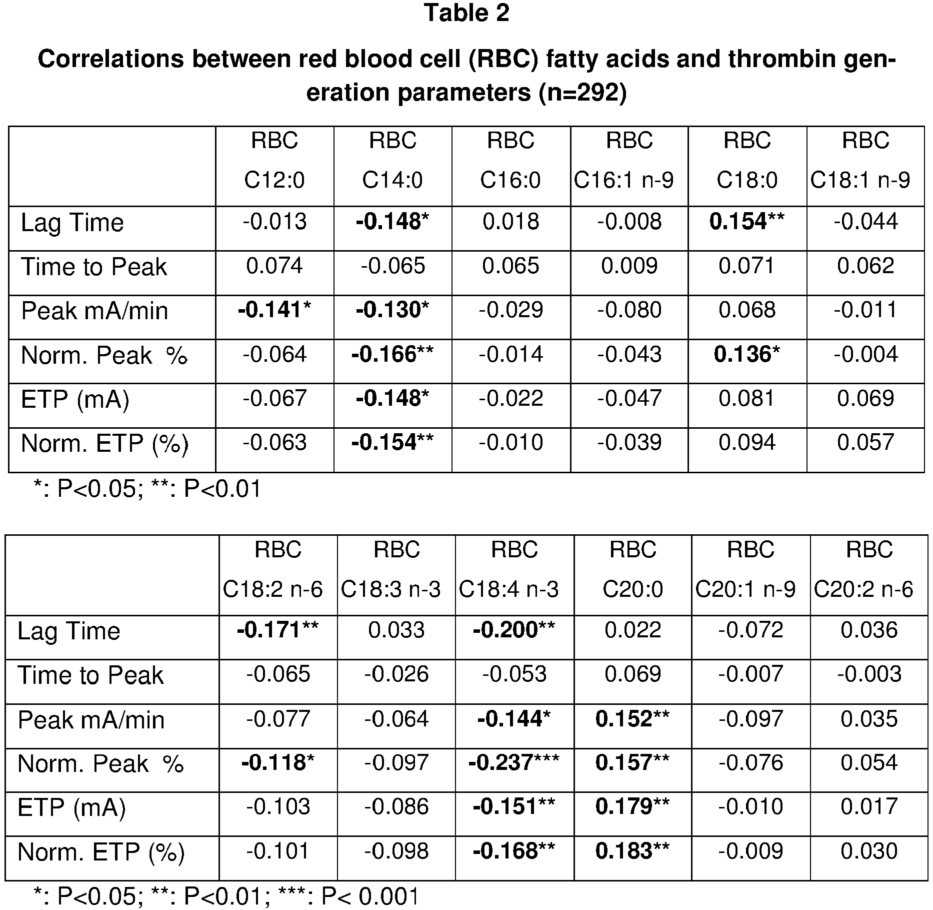

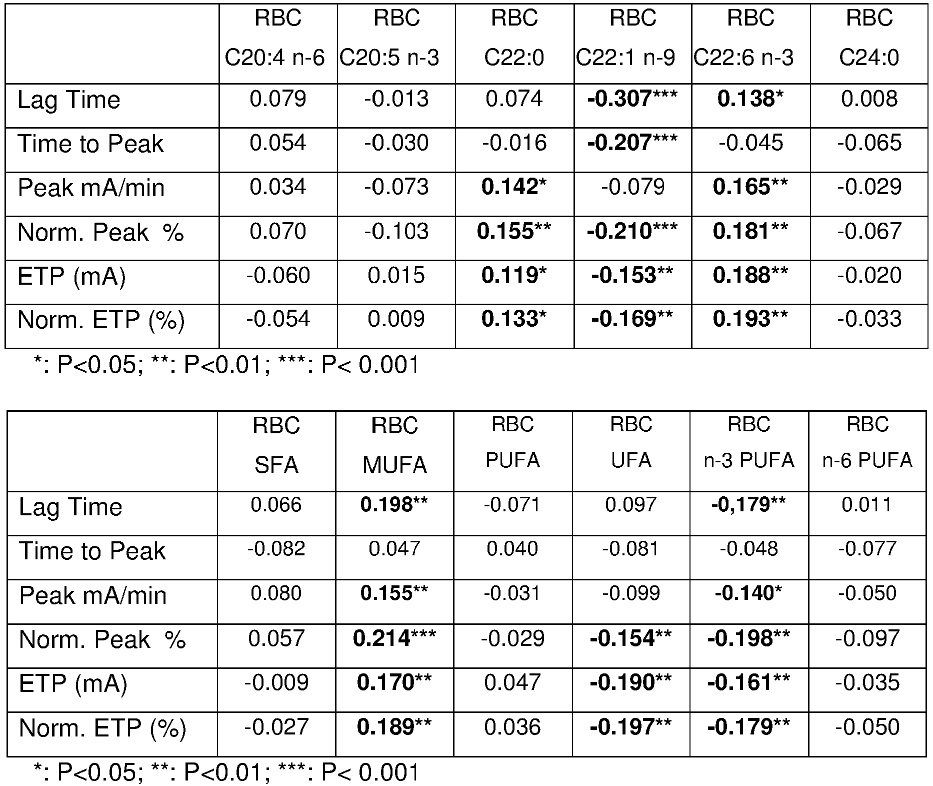

Moreover, it is noteworthy to note that in linear regression models erucic acid remained a significant predictor of thrombin generation parameters, including the endogenous thrombin potential, even after correction for all other fatty acids that showed significant correlations to the univariate analysis, as well as after correction by sex, age and diagnosis of coronary artery disease (data not shown).

### High levels of erucic acid are associated with a decreased risk of choronary atherosclerosis disease

To demonstrate the hypothesis underlying the present invention, studies have been conducted in a larger sample of the population, which also includes subjects with completely normal coronary arteries for comparison purposes, as reported above. Specifically, erucic acid levels were analysed in a sample of 1,007 subjects (mean age 60.0 ± 10.7 years, 22.9% females) with or without coronary artery disease demonstrated angiographically (312 without CAD and 695 with CAD).

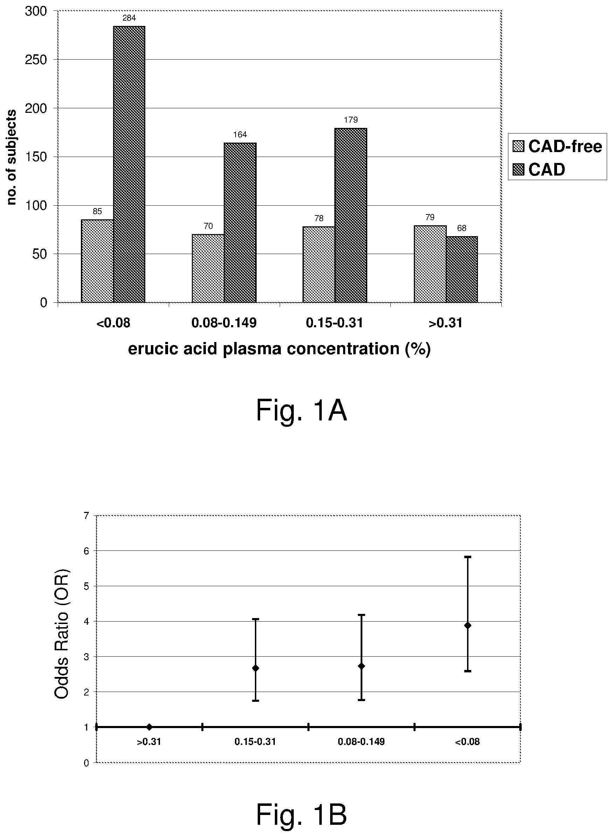

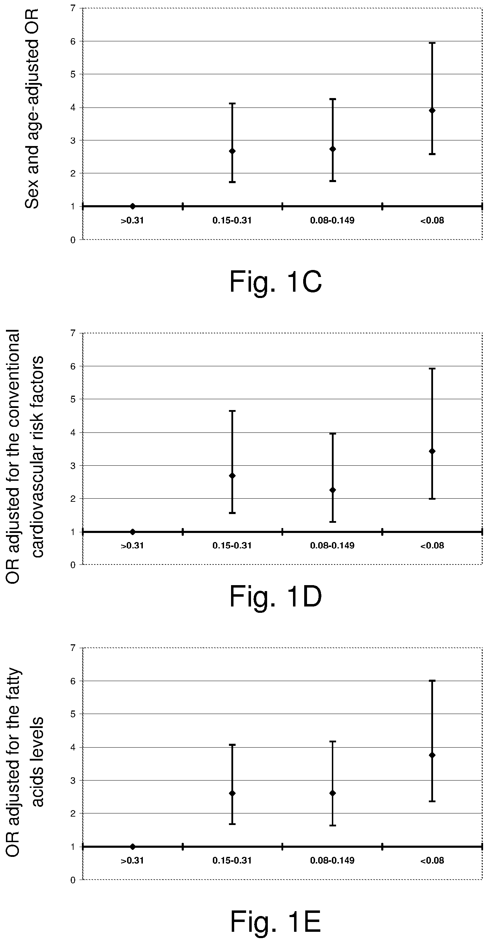

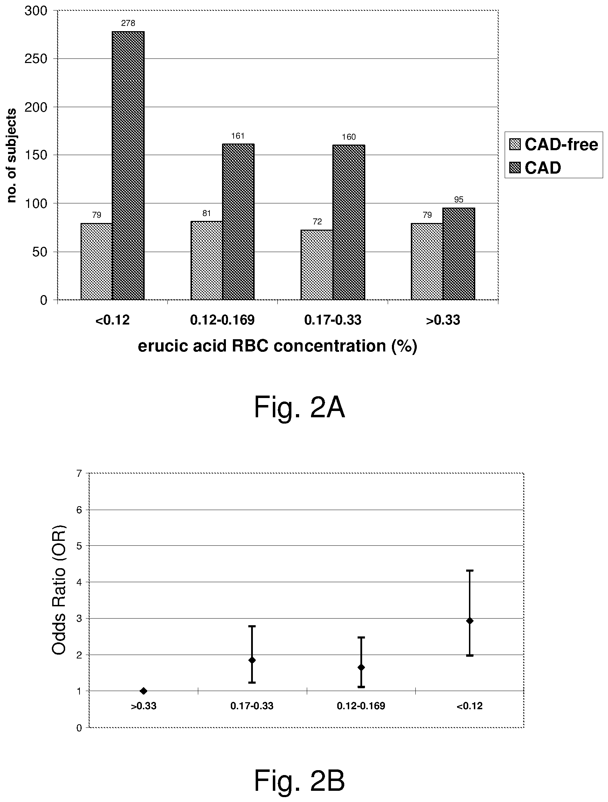

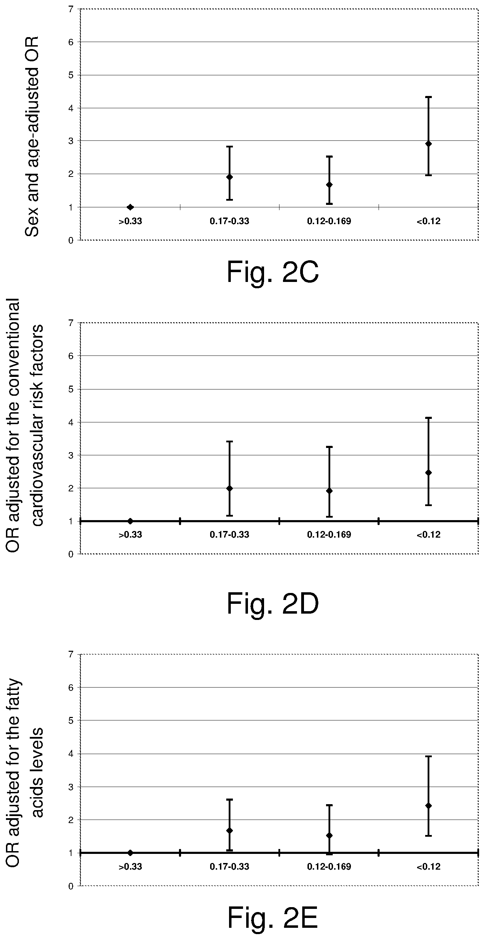

It was found that patients with CAD had lower levels of erucic acid concentrations in both plasma and erythrocytes. But what is more remarkable is that by stratifying the population on the basis of quartiles of erucic acid levels (calculated on the basis of values in the CAD-free "control" population), as shown in Figures 1 and 2, the prevalence of patients with Atherosclerotic coronary disease progressively decreased by increasing erucic acid levels, with the lowest prevalence of CAD in the highest quartile of erucic acid in plasma.

As shown in **Figure 1**, subjects belonging to the lowest quartile of erucic acid in plasma had an about four-fold increased risk of CAD than those in the highest quartile. This association remained significant after adjustment based on the sex and age of the subjects, based on all the traditional cardiovascular factors and on the basis of the other plasma fatty acids that showed a significant correlation with the plasma levels of erucic acid (data not shown). The inverse association of erucic acid levels with coronary pathologies was also confirmed by the analysis on erythrocyte fatty acids, although slightly less marked (Figure 2).

### High levels of erucic acid are associated with a decreased risk of total and cardiovascular mortality

Within the study population with known atherosclerotic coronary artery disease, 605 patients were followed prospectively for total and cardiovascular mortality: the subject's status was determined by searching National Population Register and by an ambulatory or telephone survey. Certification and date of death were obtained from the National Population Register. The causes of death were obtained from death certificates kept at the Italian Institute of Statistics (ISTAT) (Martinelli N et al. J Thromb Haemost 2016). During the study, with a median follow-up period of 57 months, 96 patients (15.9%) died, with 69 (11.4%) events due to cardiovascular causes. Death from cardiovascular causes was defined as death due to ischemic heart disease, heart failure, peripheral vascular disease or cerebrovascular disease.

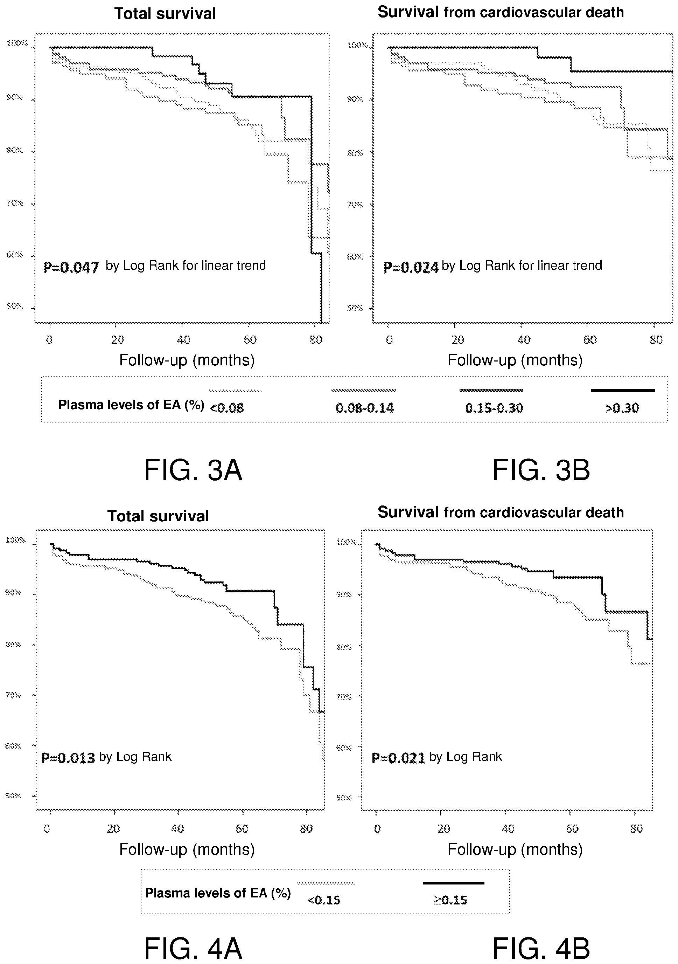

The Kaplan-Meier survival curves of **Figures 3A** and **3B** respectively show the total and cardiovascular mortality according to the already defined quartiles of erucic acid in plasma. As can be seen, patients with CAD belonging to quartiles with lower plasma erucic acid levels had a higher total and cardiovascular mortality rate than those in quartiles with higher erucic acid levels.

Considering the median value of plasma erucic acid level (0.15%) as the threshold level, **Figures 4A** and **4B** show that subjects with plasma erucic acid higher than the median value (≥0.15%) had a reduced rate of total mortality (12.1 % versus 18.2%, P = 0.013 for Log Rank) and cardiovascular mortality (8.2% against 13.4%, P = 0.021 for Log Rank) lower than subjects with lower plasma erucic acid at the median value.

The above has been confirmed in different Cox regression models adjusted for sex and age, as well as for traditional cardiovascular risk factors and other predictors of mortality due to univariate analysis (i.e. body mass index, history of myocardial infarction, grade of CAD, type of therapeutic approach, hypertension, diabetes, renal function (MDRD), high levels of plasma lipids and high levels of C-reactive protein (hs-CRP)) on subjects with plasma erucic acid higher than the median value (≥ 0.15%), which have a substantially halved risk of total and cardiovascular mortality (data not shown).

With regard to erythrocyte erucic acid, subjects within the lowest quartile (<0.12%) had a slight but not significant increase in total mortality (18.6% against 14.2%, P = 0.092 for Log Rank), while there was a significant increase in cardiovascular mortality (14.3% against 9.6%, P = 0.045 for Log Rank).

### Results from bench: erucic acid-enriched liposomes in plasma can reduce the generation of thrombin, the main procoagulant enzyme

### Methods

### Erucic acid-containing liposomes

Specific liposome-based preparations were obtained (phospholipids with a concentration of 4-10 mM, 10% of phosphatidylserine (PS) and 90% of phosphatidylcholine (PC)) in which the phosphatidylcholine could have 2 stearic acid chains or, in alternative, 2 chains of erucic acid. The two synthetic phosphatidylcholines (Avanti Polar Lipids, Inc., USA) had been diesterified with 2x stearic acid for the control PC, and with 2x erucic acid for the PC containing the active ingredient, introduced at very small percentages (0.5% and 0.1%). The two PCs were mixed in creating the liposomes.

Three different types of liposomes were produced: i) not containing erucic acid (liposome A), ii) containing 0.1% erucic acid (liposome B) and iii) containing 0.5% erucic acid (liposome C). The erucic acid contents were selected based on the plasma and erythrocytic concentrations of erucic acid observed in the population of the clinical study presented above.

**Table 3**

| Composition of liposomes preparations in (% by weight) | | | |
|---|---|---|---|
| | **A** | **B** | **C** |
| Distearoyl-phosphatidylcholine (PC(18:0,18:0) (1,2-distearoyl-sn-glycero-3-phosphocholine) | 90 | 89,9 | 89,5 |
| Distearoyl-phosphatidylserine (PS(18:0,18:0) (1,2-distearoyl-sn-glycero-3-phosphoserine) | 10 | 10 | 10 |
| Dierucoil-phosphatidylcholine (PC(22:1 n-9,22:1 n-9) (1,2-dierucoyl-sn-glycero-3-phosphocholine) | 0 | 0,1 | 0,5 |

Liposomes were extruded to obtain 200 nm homogenous diameter.

### Thrombin generation in plasma with erucic acid-containing liposomes

Thrombin generation was evaluated as reported above, adding a specific fluorogenic substrate for thrombin (Calbiochem-Novobiochem, La Jolla, CA, USA) in a pooled normal plasma (Hyphen Bio-Med) or in a mixture (1: 4 v/v) of normal plasma and of factor V depleted plasma (George King, BioMedical Inc., USA). Specific assays of thrombin generation are performed in states established in pools of normal commercial plasma, supplemented with liposomes containing or not erucic acid as per Table 3.

The plasma samples were diluted (1/20) in an HBS buffer (Hepes 20 mM, NaCl 150 mM, PEG-8000 0.1%, pH 7.4) and incubated for 5 minutes at 37°C. The reaction was triggered by the addition of a mixture with Innovin (Dade Innovin^{®}, Siemens Healthcare, Marburg, Germany) as a source of tissue factor (~8.5 pM), the liposome preparation (100 µM), CaCl₂ (2.5 mM) and the fluorogenic substrate (250 µM). The essay is summarized in the following diagram.

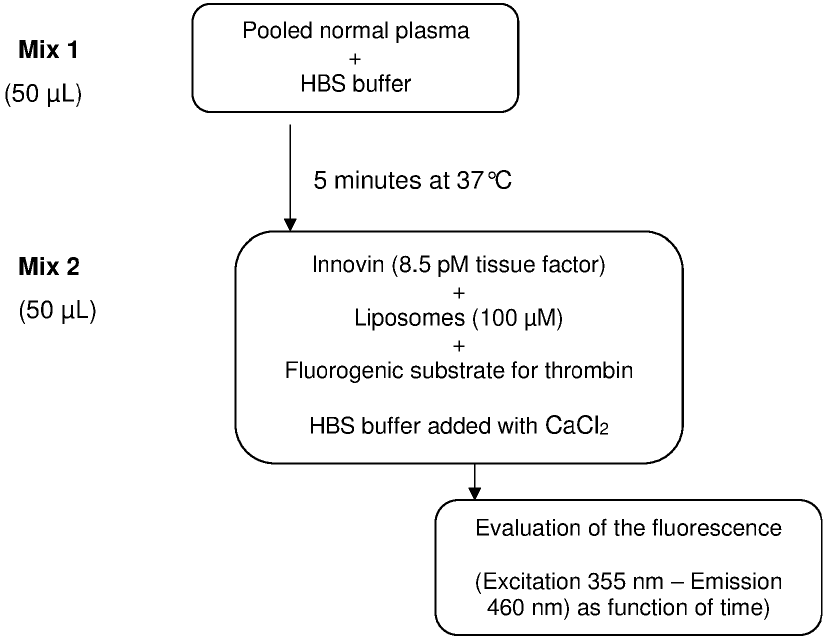

Fluorescence (excitation at 355 nm, emission of 460 nm) was measured over time with a microplate fluorometer (Fluoroskan Ascent FL, Thermo Fisher Scientific, Helsinki, Finland).

The specific parameters of thrombin generation, Lag time, time to peak (TTP), peak height (Peak) and area under the curve (Area) were obtained by a non-linear regression analysis of the first derivative of relative fluorescence units (Rfu), using the Graph-Pad Prism 5 statistical software (GraphPad, Inc., California, USA). The fluorescence generation rate (Rate) was obtained by the formula: Pic-co/(TTP - Lag time).

All experiments were performed in duplicate.

### Results

Thrombin generation in a pooled normal plasma, activated by tissue factor 8.5 pM, was significantly modified by the addition of small amounts of liposomes containing erucic acid, as indicated by the different assay parameters of the numerically illustrated test in the following table.

**Table 4**

| Thrombin generation curves in normal plasma containing liposomes A, B or C | | | | | | |
|---|---|---|---|---|---|---|
| | A (erucic acid 0 µM) | | B (erucic acid 0,1 µM) | | C (erucic acid 0,5 µM) | |
| Lag time (s) | 549 | 548 | 634 | 630 | 757 | 770 |
| TTP (s) | 935 | 935 | 1045 | 1035 | 1225 | 1215 |
| Peak (Rfu/s) | 5.0 | 4.9 | 4.8 | 4.9 | 4.2 | 4.1 |
| Area (Rfu) | 2817 | 2782 | 2735 | 2894 | 2605 | 2539 |
| Rate (Rfu/s²) | 0.013 | 0.013 | 0.012 | 0.012 | 0.009 | 0.009 |

As can be seen, exposure to liposomes with erucic acid at a concentration of 0.5 µM (liposome C) prolonged lag time (139.2 ± 1.2%) and TTP (130.5 ± 0, 6%) and decreased thrombin peak (83.8 ± 1.7%), Area (91.9 ± 1.8%) and fluorescence generation rate (Rate) (71.0 ± 1, 9%), compared to the thrombin generation parameters evaluated in the presence of liposomes without erucic acid (liposome A). Intermediate values were detected for the erucic acid concentration of less than 0.1 µM (liposome B).

The differences between all the thrombin generation parameters were statistically significant, with the exception of the Area.

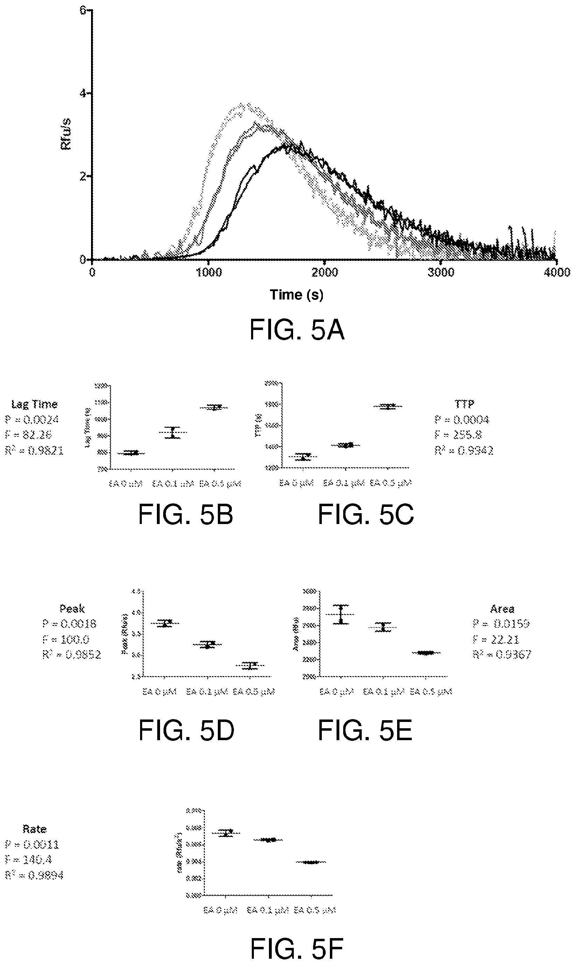

The assay was also applied to evaluate the inhibitory effect of erucic acid using a lower plasma concentration of coagulation factor V (FV) than normal (1.75 µg / mL, 25% of the normal level), which is essential for the efficiency of the prothrombinase complex. The results are shown graphically in **Figure 5** and numerically in the following table.

**Table 5**

| Thrombin generation curves in plasma depleted of coagulation factor V containing liposomes A, B or C | | | | | | |
|---|---|---|---|---|---|---|
| | A (erucic acid 0 µM) | | B (erucic acid 0,1 µM) | | C (erucic acid 0,5 µM) | |
| Lag time (s) | 804 | 789 | 896 | 943 | 1080 | 1062 |
| TTP (s) | 1325 | 1285 | 1405 | 1425 | 1795 | 1765 |
| Peak (Rfu/s) | 3.7 | 3.8 | 3.3 | 3.2 | 2.8 | 2.7 |
| Area (Rfu) | 2652 | 2805 | 2614 | 2547 | 2271 | 2289 |
| Rate (Rfu/s²) | 0.007 | 0.008 | 0.006 | 0.007 | 0.004 | 0.004 |

In this case, the effect of exposure to C liposome was comparable to that obtained at 100% FV levels, and produced a remarkable reduction in lag time (134.5 ± 1.2%) and TTP (136.4 ± 1.2%), as well as a significant decrease in Peak (74.4 ± 2.9%), Area (83.6 ± 0.6%) and Rate (53, 3 ± 1.7%).

All of these results are consistent with the previously shown epidemiological data and indicate that the presence of erucic acid in the plasma, inserted in liposomes at concentrations comparable to those measured in normal subjects, is effective in reducing thrombin generation, and has anti-coagulant properties.

The present invention has been described with reference to some of its specific embodiments, but it is to be understood that variations and modifications may be made to it by those skilled in the art without departing from the scope of the invention as defined in the appended claims.

## Claims

1. A preparation for systemic administration based on erucic acid (i.e., cis-13-docosenoic acid, 22:1 n-9) for use as an anticoagulant and antithrombotic agent.

2. The preparation for systemic administration for the use according to claim 1, in the prevention and treatment of cardiovascular diseases and thrombotic disorders.

3. The preparation for systemic administration for the use according to claims 1 or 2, wherein said cardiovascular diseases are coronary artery disease.

4. The preparation for systemic administration for the use according to any one of the previous claims, which preparation is formulated in a vehicle suitable for oral administration.

5. The preparation for systemic administration for the use according to any one of the previous claims, wherein said vehicle is made up of lipid micelles or liposomes containing erucic acid in their composition.

6. The preparation for systemic administration for the use according to claim 5, wherein said vehicle consists of liposomes containing from 0.05% to 1.00% by weight of phosphatidylcholine diesterified with erucic acid.

7. The preparation for systemic administration for the use according to claim 6, wherein said vehicle consists of liposomes containing from 0.10% to 0.50% by weight of phosphatidylcholine diesterified with erucic acid.

8. The preparation for systemic administration for the use according to any one of the previous claims, wherein said erucic acid is administered at a dosage ranging from 50 mg/day to 500 mg/day.

## Patentansprüche

1. Eine Zubereitung zur systemischen Verabreichung auf Basis von Erucasäure (d.h., cis-13-Dokosensäure, 22:1 n-9) zur Verwendung als Gerinnungshemmer und Antithrombose-Mittel.

2. Das Präparat zur systemischen Verabreichung zur Verwendung nach Anspruch 1 zur Vorbeugung und Behandlung von Herz-Kreislauf-Erkrankungen und thrombotischen Erkrankungen.

3. Das Präparat zur systemischen Verabreichung zur Verwendung nach Anspruch 1 oder 2, wobei es sich bei den Herz-Kreislauf-Erkrankungen um koronare Herzkrankheiten handelt.

4. Das Präparat zur systemischen Verabreichung zur Verwendung nach einem der vorhergehenden Ansprüche, wobei das Präparat in einem für die orale Verabreichung geeigneten Vehikel formuliert ist.

5. Das Präparat zur systemischen Verabreichung zur Verwendung nach einem der vorhergehenden Ansprüche, wobei der Träger aus Lipidmizellen oder Liposomen besteht, die in ihrer Zusammensetzung Erucasäure enthalten.

6. Präparat zur systemischen Verabreichung zur Verwendung nach Anspruch 5, wobei der Träger aus Liposomen besteht, die 0,05 bis 1,00 Gew.-% mit Erucasäure diesterisiertes Phosphatidylcholin enthalten.

7. Präparat zur systemischen Verabreichung zur Verwendung nach Anspruch 6, wobei der Träger aus Liposomen besteht, die 0,10 bis 0,50 Gew.-% mit Erucasäure diesterisiertes Phosphatidylcholin enthalten.

8. Das Präparat zur systemischen Verabreichung zur Verwendung nach einem der vorhergehenden Ansprüche, wobei die Erucasäure in einer Dosierung im Bereich von 50 mg/Tag bis 500 mg/Tag verabreicht wird.

## Revendications

1. Préparation pour administration systémique à base d'acide érucique (c'est-à-dire acide cis-13-docosénoïque, 22:1 n-9) à utiliser comme agent anticoagulant et antithrombotique.

2. Préparation pour administration systémique pour l'utilisation selon la revendication 1, dans la prévention et le traitement des maladies cardiovasculaires et des troubles thrombotiques.

3. Préparation pour administration systémique pour l'utilisation selon les revendications 1 ou 2, dans laquelle lesdites maladies cardiovasculaires sont des maladies des artères coronaires.

4. Préparation pour administration systémique pour l'utilisation selon l'une quelconque des revendications précédentes, cette préparation étant formulée dans un véhicule adapté à l'administration orale.

5. Préparation pour administration systémique pour l'utilisation selon l'une quelconque des revendications précédentes, dans laquelle ledit véhicule est constitué de micelles lipidiques ou de liposomes contenant de l'acide érucique dans leur composition.

6. Préparation pour administration systémique pour l'utilisation selon la revendication 5, dans laquelle ledit véhicule consiste en liposomes contenant de 0,05% à 1,00% en poids de phosphatidylcholine diestérifiée avec de l'acide érucique.

7. Préparation pour administration systémique pour l'utilisation selon la revendication 6, dans laquelle ledit véhicule consiste en liposomes contenant de 0,10% à 0,50% en poids de phosphatidylcholine diestérifiée avec de l'acide érucique.

8. Préparation pour administration systémique pour l'utilisation selon l'une des revendications précédentes, dans laquelle l'acide érucique est administré à un dosage allant de 50 mg/jour à 500 mg/jour.
